# FASCICULE DE BREVET EUROPEEN

(11) **EP 2 021 987 B1**
(45) Date de publication et mention de la délivrance du brevet: **17.08.2016**
(21) Numéro de dépôt: 07731929.1
(22) Date de dépôt: 23.04.2007
(51) Int. Cl.: G06M 1/24, G06M 1/04, A61M 15/00

(54) **INDICATEUR DE DOSES POUR DISPOSITIF DE DISTRIBUTION DE PRODUIT FLUIDE**
DOSISINDIKATOR FÜR EINE FLUIDPRODUKTABGABEEINRICHTUNG
DOSE INDICATOR FOR A FLUID PRODUCT DISPENSING DEVICE

(30) Priorité: 24.04.2006 FR 0651425
(43) Date de publication de la demande: 11.02.2009
(73) Titulaire: Aptar France SAS, 27110 Le Neubourg (FR)
(72) Inventeur: STRADELLA, Fabio, 16032 Camogli (IT); STRADELLA, Giuseppe, 16032 Camogli (IT)
(74) Mandataire: CAPRI
(86) Numéro de dépôt international: PCT/FR2007/051155
(87) Numéro de publication internationale: WO 2007/122358

(56) Documents cités:
- EP-A1- 1 386 630
- WO-A-98/52634
- WO-A-03/028792
- WO-A2-2005/079727
- FR-A1- 2 857 770

## Description

La présente invention concerne un indicateur de doses, ainsi qu'un dispositif de distribution de produit fluide comportant un tel indicateur.

Dans le domaine des dispositifs de distribution de produit fluide destinés à distribuer plusieurs doses, et en particulier dans le domaine des pulvérisateurs, de nombreux systèmes destinés à indiquer le nombre de doses distribuées ou le nombre de doses restant à distribuer ont été développés.

La plupart de ces systèmes présentent de nombreux inconvénients. Ainsi, ils sont généralement conçus aux moyens de nombreuses pièces constitutives coopérant les unes avec les autres. Par conséquent, ces compteurs ou indicateurs peuvent devenir très complexes, encombrants, et donc coûteux à fabriquer et à assembler. Les documents EP-1 386 630 et WO 2005/079727 décrivent de tels systèmes complexes. De plus, les systèmes d'indication comportent souvent des éléments élastiquement déformables pour l'actionnement du système. Dans certains cas, ces éléments peuvent se déformer après plusieurs actionnements, et alors ne plus garantir un fonctionnement fiable du système jusqu'à la dernière dose. Le document WO 98/52634 décrit un tel système. D'autre part, l'indication est généralement donnée par des chiffres, qui sont souvent difficiles à lire pour l'utilisateur, en particulier lorsque les dispositifs de distribution sont destinés à distribuer un grand nombre de doses, par exemple jusqu'à 200 doses. Un autre inconvénient majeur réside dans le fait que les compteurs existants nécessitent généralement une procédure d'assemblage du dispositif de distribution qui est modifiée de par la présence du compteur, et qui diffère donc de la procédure d'assemblage habituelle. Ceci augmente la complexité du dispositif, et implique par conséquent un coût supérieur.

D'autre part, une exigence de sécurité très important est d'éviter tout risque de sous comptage, c'est-à-dire le fait de ne pas compter une distribution totale ou partielle de produit. Pour éviter ce risque, il est nécessaire que l'actionnement du compteur soit réalisé pendant la course de l'organe de distribution, en particulier de la soupape de valve, qui se produit avant le début de l'expulsion du produit. La longueur de cette course initiale est généralement très courte, typiquement de l'ordre de 1 à 1,5 mm, et les diverses tolérances de dimensions du dispositif réduisent celle-ci à quelques dixièmes de millimètres. Une course d'actionnement aussi courte rend difficile l'actionnement du compteur et peut impliquer l'utilisation de mécanismes complexes pour garantir un comptage fonctionnel.

La présente invention a pour but de fournir un indicateur de doses destiné à un dispositif de distribution de produit fluide, qui ne reproduit pas les inconvénients susmentionnés.

En particulier, la présente invention a pour but de fournir un indicateur de doses qui est simple et peu coûteux à fabriquer et à assembler, de fonctionnement fiable jusqu'à la dernière dose et qui peut notamment être appliqué à tous les dispositifs de distribution de produit fluide existants sans impliquer une modification importante de la procédure d'assemblage.

La présente invention a aussi pour but de fournir un indicateur de doses constitué d'un faible nombre de pièces constitutives, même si le nombre de doses contenu dans le dispositif de distribution est élevé, par exemple 200 doses.

La présente invention a également pour but de fournir un indicateur de doses qui forme une unité complète et séparée, et qui comprend notamment les moyens d'actionnement de l'indicateur.

La présente invention a aussi pour but de fournir un indicateur de doses qui soit facile à lire pour l'utilisateur.

La présente invention a encore pour but de fournir un indicateur de doses qui évite tout risque de sous comptage (non prise en compte d'une dose distribuée). Plus particulièrement, la présente invention à pour but de fournir un indicateur de doses qui compte dès le début de la course d'actionnement du dispositif de distribution auquel il est associé, même si cette course est très courte.

La présente invention a donc pour objet un indicateur de doses pour indiquer le nombre de doses distribuées ou restant à distribuer à partir d'un dispositif de distribution de produit fluide, caractérisé en ce qu'il comporte un corps d'indicateur, un élément d'actionnement, un élément rotatif de transmission, un premier élément de comptage rotatif et un second élément de comptage rotatif, lesdits premier et second éléments de comptage rotatif étant disposés côte à côte dans un même plan et tournant autour d'axes de rotation parallèles, lesdits premier et second éléments de comptage coopérant pour fournir une indication commune à chaque actionnement de l'indicateur, ledit premier élément de comptage rotatif étant un disque comportant une surface supérieure et une surface inférieure, ladite surface supérieure comportant des premiers moyens d'indication disposés au niveau du bord périphérique externe de ladite surface supérieure et ladite surface inférieure comportant un premier engrenage coopérant avec un engrenage d'actionnement prévu sur ledit élément rotatif de transmission, ledit premier élément de comptage rotatif comportant une dent d'entraînement adaptée à coopérer à chaque tour complet dudit premier élément de comptage rotatif avec un second engrenage prévu sur ledit second élément de comptage rotatif.

Avantageusement, lesdits premiers moyens d'indication comportent des chiffres, notamment les chiffres 0 à 9.

Avantageusement, le second élément de comptage rotatif est un disque comportant une surface supérieure et une surface inférieure, ladite surface supérieure comportant des seconds moyens d'indication disposés au niveau du bord périphérique externe de ladite surface supérieure.

Avantageusement, lesdits seconds moyens d'indication comportent des chiffres, notamment les chiffres 00 à 20.

Avantageusement, ladite surface inférieure dudit second élément de comptage rotatif comporte un second engrenage coopérant avec une dent d'entraînement dudit premier élément de comptage rotatif.

Avantageusement, ledit second élément de comptage rotatif comporte une denture de positionnement coopérant avec une patte élastique dudit élément d'actionnement, notamment pour assurer un positionnement précis dudit second élément de comptage rotatif à chacune de ses rotations.

Avantageusement, ledit second élément de comptage rotatif comporte des moyens de butée pour bloquer l'actionnement de l'indicateur après un nombre prédéterminé d'indications, notamment 200.

Avantageusement, lesdits moyens de butée comportent un profil saillant, tel qu'un ergot, solidaire dudit second élément de comptage rotatif, et coopérant avec une partie fixe du corps de l'indicateur.

Avantageusement, ledit élément rotatif de transmission comporte un premier engrenage d'actionnement coopérant avec ledit élément d'actionnement et un second engrenage d'actionnement coopérant avec un premier engrenage dudit premier élément de comptage rotatif.

Avantageusement, ledit élément rotatif de transmission est un disque comportant une surface supérieure et une surface inférieure, ladite surface inférieure comportant ledit premier engrenage d'actionnement et ladite surface supérieure comportant ledit second engrenage d'actionnement.

Avantageusement, ledit élément d'actionnement comporte des moyens anti-retour telle qu'une patte élastique, coopérant avec ledit élément rotatif de transmission pour empêcher une rotation de celui-ci dans le sens opposé au sens de rotation lors d'un actionnement de l'indicateur.

Avantageusement, ledit élément d'actionnement comporte une première patte élastique coopérant avec un premier engrenage d'actionnement de l'élément rotatif de transmission, pour faire tourner ledit élément rotatif de transmission dans le sens d'actionnement à chaque actionnement de l'indicateur, une seconde patte élastique anti-retour, coopérant avec ledit premier engrenage d'actionnement de l'élément rotatif de transmission pour éviter toute rotation dudit élément rotatif de transmission en sens inverse audit sens d'actionnement, et une troisième patte élastique coopérant avec une denture de positionnement dudit second élément de comptage rotatif, pour assurer un positionnement angulaire précis dudit second élément de comptage rotatif à chacune de ses rotations.

Avantageusement, ledit élément d'actionnement comporte une partie fixe par rapport au corps de l'indicateur et une partie déformable, adaptée à coopérer avec une partie d'actionnement d'un dispositif de distribution de produit fluide, ladite partie d'actionnement étant mobile par rapport audit élément d'actionnement sur une course d'actionnement.

Avantageusement, ladite partie déformable dudit élément d'actionnement comporte une première partie déformable ayant une première flexibilité, et une seconde partie déformable ayant une seconde flexibilité, ladite seconde flexibilité étant inférieure à ladite première flexibilité.

Avantageusement, ladite première partie déformable réalise l'actionnement de l'indicateur, et ladite seconde partie déformable plus rigide permet la poursuite de la course d'actionnement de ladite partie du dispositif de distribution de produit fluide, après actionnement dudit indicateur.

Avantageusement, l'actionnement dudit indicateur a lieu au tout début de la course d'actionnement dudit dispositif de distribution de produit fluide.

Avantageusement, ladite partie d'actionnement du dispositif de distribution de produit fluide, qui coopère avec la partie déformable de l'élément d'actionnement, est un réservoir de produit fluide, ledit corps de l'indicateur étant fixe par rapport au corps du dispositif de distribution de produit fluide.

Avantageusement, ladite partie d'actionnement du dispositif de distribution de produit fluide, qui coopère avec la partie déformable de l'élément d'actionnement, est un corps dudit dispositif de distribution de produit fluide, ledit corps de l'indicateur étant fixe par rapport à un réservoir de produit fluide.

Avantageusement, ledit corps de l'indicateur comporte une fenêtre de visualisation permettant d'afficher l'indication commune desdits premier et second éléments de comptage rotatifs.

La présente invention a aussi pour objet un dispositif de distribution de produit fluide comportant un corps, un réservoir mobile sur une course d'actionnement par rapport audit corps et un embout d'inhalation, ainsi qu'un indicateur tel que décrit ci-dessus.

Ces caractéristiques et avantages et d'autres de la présente invention apparaîtront plus clairement au cours de la description détaillée suivante d'un mode de réalisation particulier de celle-ci, faite en référence aux dessins joints, donnés à titre d'exemples non limitatifs, et sur lesquels,
- la figure 1 est une vue schématique de côté d'un dispositif de distribution de produit fluide (B) comportant un indicateur de doses (A) selon un mode de réalisation avantageux de la présente invention,
- la figure 2 est une vue schématique en perspective d'une partie d'un indicateur de doses selon un mode de réalisation avantageux de l'invention,
- la figure 3 est une vue similaire à celle de la figure 2, selon un autre angle de vue,
- la figure 4 est une vue similaire à celle de la figure 3, prise selon encore un autre angle de vue,
- la figure 5 est une vue schématique de dessus des deux éléments de comptages rotatifs, montrant une indication commune de 200,
- la figure 6 est une vue similaire à celle de la figure 5, vue de dessous, et
- la figure 7 est une vue schématique en perspective de l'élément rotatif de transmission et du premier élément de comptage rotatif.

L'indicateur de doses A de la présente invention s'applique à tous types de dispositifs de distribution de produit fluide. Il s'applique toutefois plus particulièrement aux dispositifs de pulvérisation, et avantageusement aux dispositifs d'inhalation aérosols comportant une valve doseuse montée sur un récipient contenant un produit et un gaz propulseur.

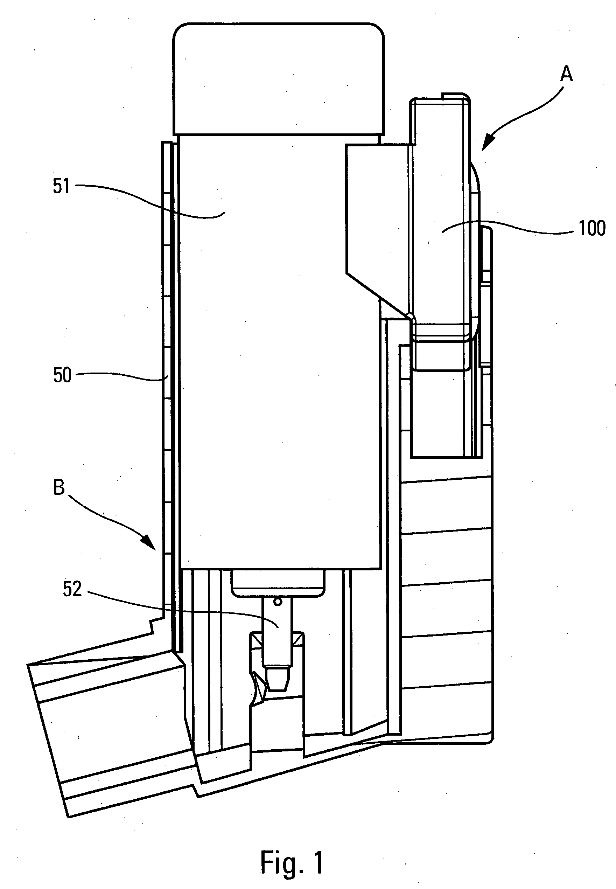

La figure 1 représente schématiquement un dispositif de distribution B auquel l'indicateur de doses A de la présente invention est particulièrement adapté. Ce dispositif comporte un corps 50 et un réservoir 51 sur lequel est assemblé une valve doseuse 52. L'actionnement du dispositif B étant obtenu par déplacement axial du réservoir 51 à l'intérieur du corps 50 sur une course d'actionnement, ce déplacement entraînant une compression de la soupape de la valve 52 ce qui provoque l'expulsion d'une dose de produit à travers un orifice buccal 55. Bien entendu, la présente invention s'applique également à d'autres types de dispositifs de distribution, et notamment des dispositifs de pulvérisation du type nasal, ou des dispositifs comportant une pompe en lieu et place de la valve. Dans l'exemple représenté, l'indicateur A est fixé au réservoir 51, d'une quelconque manière appropriée. Il pourrait toutefois être positionné différemment ou être fixé au corps 50 du dispositif.

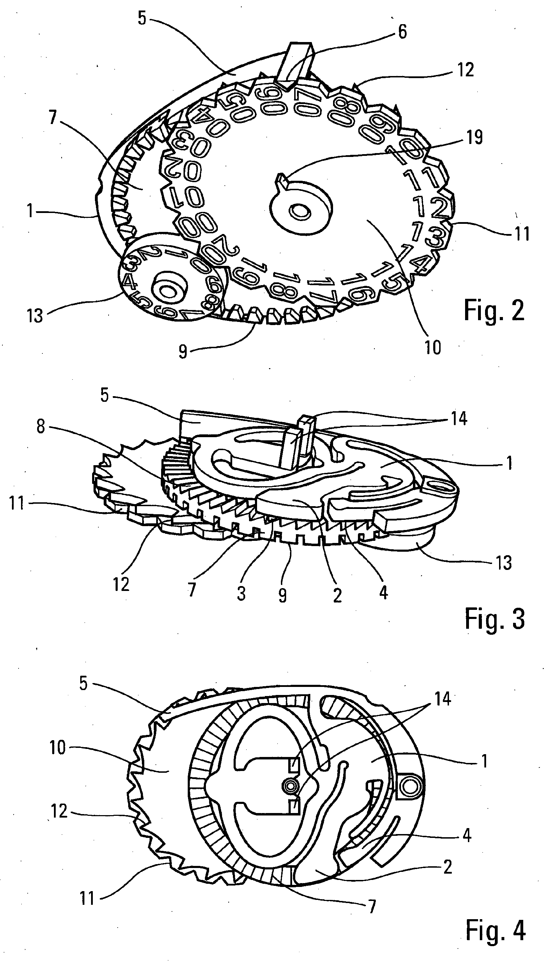

Les figures 2 à 7 représentent un indicateur de doses A qui peut notamment être utilisé avec un dispositif de distribution de produit fluide B décrit ci-dessus. Cet indicateur de doses comprend un corps d'indicateur 100 dans lequel sont contenus un élément d'actionnement 1, un élément rotatif de transmission 7, un premier élément de comptage rotatif 13 et un second élément de comptage rotatif 10. Le corps d'indicateur 100 comporte avantageusement un couvercle qui incorpore une fenêtre de visualisation à travers laquelle l'utilisateur peut visualiser l'indication de l'indicateur A. L'indicateur de la présente invention comporte donc seulement quatre pièces déformables et/ou mobiles.

Selon l'invention, le premier élément de comptage rotatif 13 et le second élément de comptage rotatif 10 sont disposés côte à côte dans un même plan et tournent autour d'axes de rotation parallèles de telle sorte que lesdits premier et second éléments de comptage rotatifs 13, 10 coopèrent pour fournir une indication commune à chaque actionnement.

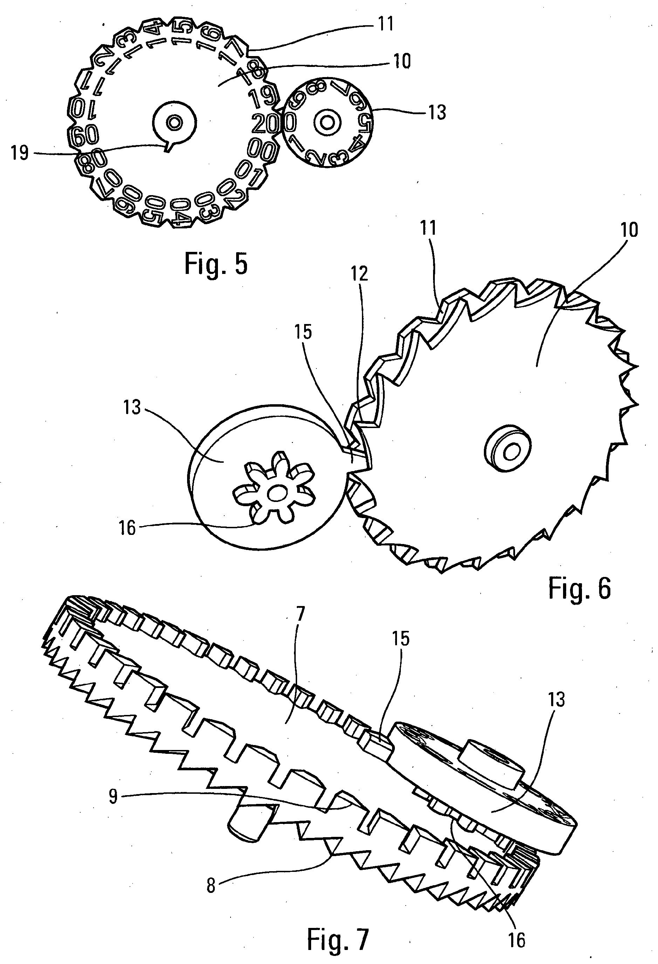

La figure 5 représente en particulier la position de départ d'un indicateur adapté à compter le nombre de doses restant à l'intérieur du réservoir 51, avec un nombre maximal de doses égal à 200. Ainsi, en se référant à cette figure 5, l'indication commune fournie par les premier et second éléments de comptage rotatif 13, 10 forme le chiffre 200, qui est alors visible à travers le fenêtre de visualisation du corps d'indicateur 100.

Comme visible sur les figures, le premier élément de comptage rotatif 13 peut être réalisé sous la forme d'un disque comportant une surface supérieure et une surface inférieure, la surface supérieure comportant des premiers moyens d'indication, disposés au niveau du bord périphérique externe de ladite surface supérieure, alors que la surface inférieure comporte un premier engrenage 16. De manière similaire, le second élément de comptage rotatif 10 peut également être formé sous la forme d'un disque comportant une surface supérieure et une surface inférieure, ladite surface supérieure comportant des seconds moyens d'indication, disposés avantageusement au niveau du bord périphérique externe de ladite surface supérieure, et ladite surface inférieure comportant un second engrenage 12.

De préférence, lorsque l'indicateur est destiné à compter 200 doses, le premier élément de comptage rotatif 13 comporte les chiffres 0 à 9 répartis autour de sa périphérie, alors que le second élément de comptage rotatif comporte les chiffres 00 à 20, également répartis sur sa périphérie, comme visible sur la figure 5.

Avantageusement, les premier et second éléments de comptage rotatif 13, 10 tournent autour d'axes de rotation qui sont solidaires du couvercle du corps d'indicateur 100.

Derrière les premier et second éléments de comptage rotatif 13, 10, dans la direction de visualisation d'un utilisateur, est disposé l'élément rotatif de transmission 7. Cet élément rotatif de transmission 7 est également de préférence réalisé sous la forme d'un disque comportant une surface supérieure et une surface inférieure. La surface inférieure comporte de préférence un premier engrenage d'actionnement 8, et la surface supérieure comporte avantageusement un second engrenage d'actionnement 9. Ce second engrenage d'actionnement 9 est adapté à coopérer avec le premier engrenage 16 du premier élément de comptage rotatif 13. Ceci est notamment visible sur les figures 2 et 7. Ainsi, à chaque rotation de l'élément rotatif de transmission 7, correspond une rotation du premier élément de comptage rotatif 13. Comme visible notamment sur la figure 6, le premier élément de comptage rotatif 13 comporte une dent d'entraînement 15 adaptée à coopérer à chaque tour complet du premier élément de comptage rotatif 13 avec le second engrenage 12 prévu sur ledit second élément de comptage rotatif 10. Ainsi, chaque fois que le premier élément de comptage rotatif 13 fait un tour complet, il entraîne en rotation le second élément de comptage rotatif 10. Dans cette configuration, le premier élément de comptage rotatif correspond à l'indication des unités (de 0 à 9), alors que le second élément de comptage rotatif 10 correspond à une indication des dizaines (de 00 à 20). L'indicateur représenté sur les dessins permet donc de compter 200 doses.

Avantageusement, le second élément de comptage rotatif 10 comporte en outre des moyens de butée 19, notamment réalisés sous la forme d'un profil saillant, tel qu'un ergot, et qui peut être solidaire dudit second élément de comptage rotatif 10 comme visible notamment sur la figure 2. Cet ergot 19 est adapté à coopérer avec une partie fixe du corps 100 de l'indicateur, après un nombre prédéterminé d'indications, en particulier après 200 indications dans le cas de l'indicateur représenté sur les dessins. Cet ergot 19 permet donc d'éviter que l'indicateur continue de fonctionner une fois que le nombre maximal de doses a été compté.

Derrière l'élément rotatif de transmission 7, dans le sens de visualisation d'un utilisateur, est disposé l'élément d'actionnement 1. Cet élément d'actionnement 1 coopère d'une part avec une partie mobile (par rapport audit élément d'actionnement) du dispositif de distribution de produit fluide B (voir notamment sur la figure 1), et d'autre part est adapté à entraîner en rotation ledit élément rotatif de transmission 7 à chaque actionnement du dispositif. Le fonctionnement de cet élément d'actionnement 1 sera décrit plus en détail ci-après.

De manière simplifiée, l'élément d'actionnement 1 comporte une partie fixe par rapport au corps 100 de l'indicateur A, et une partie déformable. Lorsque l'utilisateur actionne le dispositif de distribution B, notamment lorsqu'il enfonce le réservoir 51 à l'intérieur du corps 50 dans le cadre d'un dispositif B tel que représenté sur la figure 1, il provoque une déformation de la partie déformable de l'élément d'actionnement 1, cette déformation provoquant la rotation de l'élément rotatif de transmission 7 qui à son tour provoque la rotation du premier élément de comptage rotatif 13. Avantageusement, l'élément d'actionnement 1 comporte une première patte flexible 2 adaptée à coopérer avec le premier engrenage d'actionnement 8 prévu sur l'élément rotatif de transmission 7 pour faire tourner ledit élément rotatif de transmission 7 dans un sens d'actionnement à chaque actionnement de l'indicateur. Par ailleurs, l'élément d'actionnement 1 comporte également avantageusement une seconde patte flexible anti-retour 4, qui coopère de préférence également avec ledit premier engrenage d'actionnement 8 de l'élément rotatif de transmission 7 pour éviter toute rotation de cet élément rotatif de transmission 7 en sens inverse par rapport audit sens d'actionnement. Enfin l'élément d'actionnement 1 peut comporter avantageusement une troisième patte flexible 5 adaptée à coopérer avec une denture de positionnement 11 réalisée sur le second élément de comptage rotatif 10, afin d'assurer un positionnement angulaire précis dudit second élément de comptage rotatif 10 à chacune de ses rotations. Cette denture de positionnement 11 est plus précisément représentée sur la figure 2, qui montre la troisième patte flexible 5 pourvue à son extrémité d'une dent 6 venant s'engrainer dans ladite denture de positionnement périphérique 11 réalisée sur le bord latéral dudit second élément de comptage rotatif 10.

Afin de permettre la poursuite de la course d'actionnement du dispositif de distribution de produit fluide après actionnement de l'indicateur, l'élément d'actionnement 1 comporte une première partie flexible ayant une première flexibilité et une seconde partie flexible ayant une seconde flexibilité inférieure à la première flexibilité. En d'autres mots, la première partie flexible, qui incorpore la première patte flexible 2 provoquant l'actionnement de l'indicateur A, est actionnée dès que l'élément d'actionnement 1 est déformé lors de l'actionnement du dispositif. Une fois que l'indicateur a été actionné, une poursuite de la course d'actionnement est rendue possible par la seconde partie flexible plus rigide qui ne se déforme qu'après l'actionnement de l'indicateur.

L'actionnement de l'indicateur A, et en particulier la rotation de l'élément rotatif de transmission 7 est donc avantageusement réalisé par l'élément d'actionnement 1 intégré dans ledit indicateur A. Cet élément d'actionnement 1 peut comporter une première patte flexible 2 adaptée à coopérer avec ledit premier engrenage d'actionnement à chaque fois qu'une dose est distribuée, de préférence au moyen d'un organe d'actionnement 3, tel qu'une dent.

L'élément d'actionnement 1 comporte également un élément de transmission 14 qui est adapté à coopérer avec le dispositif de distribution de produit fluide B, à chaque actionnement de celui-ci. En particulier, comme visible notamment sur la figure 3, ledit élément de transmission 14 peut comprendre un ou plusieurs épaulement(s) solidaire(s) de l'élément d'actionnement 1 et qui coopère avec une partie d'actionnement 50, 51 du dispositif de distribution de produit fluide B qui est mobile pendant l'actionnement par rapport à l'élément d'actionnement 1. Dans l'exemple représenté, il s'agit d'une partie du corps 50. Bien entendu, et plus généralement, toute partie qui se déplace par rapport à l'élément d'actionnement 1 lors de l'actionnement du dispositif B peut être adaptée à coopérer avec les épaulements 14 pour actionner l'indicateur de doses A. Ainsi, si le corps 100 de l'indicateur A est fixé au réservoir 51, alors une partie fixe par rapport au corps 50 pourra coopérer avec l'élément d'actionnement 1. Au contraire, si le corps 100 de l'indicateur A est fixe par rapport au corps 50, alors le réservoir 51, ou toute partie solidaire de celui-ci, pourrait coopérer avec l'élément d'actionnement 1.

L'élément d'actionnement 1 peut être pourvu de deux parties flexibles de flexibilité différente, la première partie flexible étant plus flexible que la seconde partie flexible. Cette seconde partie plus rigide supporte lesdits épaulements 14, et lorsque le dispositif de distribution B est actionné, une partie d'actionnement du dispositif de distribution B entraîne d'abord la première partie plus flexible, comportant la première patte flexible 2, à se fléchir par rapport à l'élément rotatif de transmission 7, ce qui provoque la rotation dudit élément rotatif de transmission 7 au moyen de la dent 3 qui coopère avec le premier engrenage d'actionnement 8. La première patte flexible 2 peut aussi coopérer avantageusement avec des moyens de butée adaptés à bloquer le déplacement de la première patte flexible 2 après l'équivalent d'une dent du premier engrenage d'actionnement 8. Ainsi, pendant l'actionnement, les épaulements 14 sont déplacés par le dispositif de distribution B, et la première patte flexible 2 se fléchit jusqu'à ce que la première patte flexible 2 contacte les moyens de butée. Ceci entraîne la rotation sur l'équivalent d'une dent de l'élément rotatif de transmission 7. La première patte flexible 2 est alors bloquée, et une poursuite de la course d'actionnement du dispositif de distribution B est possible par la flexion de la seconde partie plus rigide de l'élément d'actionnement 1. De cette manière, on permet un actionnement de l'indicateur de doses A au tout début de ladite course d'actionnement. Ceci élimine tout risque de non comptage d'une dose distribuée (partiellement ou totalement) en cas d'actionnement partiel du dispositif de distribution B, tout en permettant une poursuite de la course d'actionnement après comptage. Les moyens anti-retour 4 assurent que chaque dose n'est comptée qu'une seule fois.

Le nombre de dents sur les différents engrenages donne les caractéristiques de l'indicateur de doses, et notamment le nombre de doses que cet indicateur peut compter. Le nombre maximal de doses et le mode d'affichage peuvent être variés à souhait en modifiant les moyens d'indication, ou le nombre de dents sur un ou plusieurs de ces engrenages. La présente invention permet donc de réaliser des indicateurs de doses adaptés à compter un nombre quelconque de doses, sans modifier la géométrie ou la taille dudit indicateur. Comme déjà précisé précédemment, la structure dimensionnelle du présent indicateur est particulièrement faible, notamment dans la dimension de l'épaisseur, et cet indicateur A peut donc très facilement être intégré dans les dispositifs de distribution de produit fluide B existants, comme cela est visible sur la figure 1.

L'indicateur de doses de la présente invention permet de visualiser de manière simple, peu coûteuse et progressive le nombre de doses distribuées ou le nombre de doses restant à distribuer dans le dispositif. La structure de l'indicateur est mince, indépendamment du nombre de doses qu'il doit indiquer, et il ne comporte aucune partie saillante impliquant une modification du dispositif auquel il est appliqué. Comme visible sur la figure 1, l'indicateur de doses A de la présente invention s'applique très facilement à tous les dispositifs existants, sans que ceux-ci ne doivent être modifiés. La présence de l'indicateur A ne modifie pas non plus le processus d'assemblage du dispositif B. L'indicateur peut par exemple être fixé au réservoir 51 par tous moyens appropriés. Un autre avantage du présent indicateur est que les moyens d'actionnement de l'indicateur sont intégrés dans celui-ci, de sorte que l'indicateur forme une unité autonome et séparée qui peut être pré-assemblée, et intégrée aisément dans n'importe quel dispositif de distribution de produit fluide. L'indicateur de doses de la présente invention garantit surtout l'actionnement dudit indicateur en tout début de course d'actionnement, notamment pendant la course initiale qui se produit avant le début de l'expulsion de la dose. Par ailleurs, l'indicateur de l'invention est simple et fiable, ne comportant que quatre pièces déformables et/ou rotatives.

Bien entendu, la présente invention a été décrite en référence à un mode de réalisation particulier de celle-ci, représenté sur les dessins, mais elle n'est aucunement limitée à cette forme de réalisation particulière. Au contraire, un homme du métier peut y apporter toutes modifications sans sortir du cadre de la présente invention tel que défini dans les revendications annexées.

## Revendications

1. Indicateur de doses (A) pour indiquer le nombre de doses distribuées ou restant à distribuer à partir d'un dispositif de distribution de produit fluide (B), comportant un corps d'indicateur (100), et ne comportant que quatre pièces déformables et/ou rotatives, à savoir un élément d'actionnement (1), un élément rotatif de transmission (7), un premier élément de comptage rotatif (13) et un second élément de comptage rotatif (10), lesdits premier et second éléments de comptage rotatifs (13, 10) étant disposés côte à côte dans un même plan et tournant autour d'axes de rotation parallèles, lesdits premier et second éléments de comptage (13, 10) coopérant pour fournir une indication commune à chaque actionnement de l'indicateur, ledit premier élément de comptage rotatif (13) étant un disque comportant une surface supérieure et une surface inférieure, ladite surface supérieure comportant des premiers moyens d'indication disposés au niveau du bord périphérique externe de ladite surface supérieure et ladite surface inférieure comportant un premier engrenage (16) coopérant avec un engrenage d'actionnement (9) prévu sur ledit élément rotatif de transmission (7), ledit premier élément de comptage rotatif (13) comportant une dent d'entraînement (15) adaptée à coopérer à chaque tour complet dudit premier élément de comptage rotatif (13) avec un second engrenage (12) prévu sur ledit second élément de comptage rotatif (10).

2. Indicateur selon la revendication 1, dans lequel lesdits premiers moyens d'indication comportent des chiffres, notamment les chiffres 0 à 9.

3. Indicateur selon l'une quelconque des revendication précédentes, dans lequel le second élément de comptage rotatif (10) est un disque comportant une surface supérieure et une surface inférieure, ladite surface supérieure comportant des seconds moyens d'indication disposés au niveau du bord périphérique externe de ladite surface supérieure.

4. Indicateur selon la revendication 3, dans lequel lesdits seconds moyens d'indication comportent des chiffres, notamment les chiffres 00 à 20.

5. Indicateur selon la revendication 3 ou 4, dans lequel ladite surface inférieure dudit second élément de comptage rotatif (10) comporte un second engrenage (12) coopérant avec une dent d'entraînement (15) dudit premier élément de comptage rotatif (13).

6. Indicateur selon l'une quelconque des revendications précédentes, dans lequel ledit second élément de comptage rotatif (10) comporte une denture de positionnement (11) coopérant avec une patte élastique (5) dudit élément d'actionnement (1), notamment pour assurer un positionnement précis dudit second élément de comptage rotatif (10) à chacune de ses rotations.

7. Indicateur selon l'une quelconque des revendications précédentes, dans lequel ledit second élément de comptage rotatif (10) comporte des moyens de butée (19) pour bloquer l'actionnement de l'indicateur (A) après un nombre prédéterminé d'indications, notamment 200.

8. Indicateur selon la revendication 7, dans lequel lesdits moyens de butée (19) comportent un profil saillant, tel qu'un ergot (19), solidaire dudit second élément de comptage rotatif (10), et coopérant avec une partie fixe du corps (100) de l'indicateur.

9. Indicateur selon l'une quelconque des revendications précédentes, dans lequel ledit élément rotatif de transmission (7) comporte un premier engrenage d'actionnement (8) coopérant avec ledit élément d'actionnement (1) et un second engrenage d'actionnement (9) coopérant avec un premier engrenage (16) dudit premier élément de comptage rotatif (13).

10. Indicateur selon la revendication 9, dans lequel ledit élément rotatif de transmission (7) est un disque comportant une surface supérieure et une surface inférieure, ladite surface inférieure comportant ledit premier engrenage d'actionnement (8) et ladite surface supérieure comportant ledit second engrenage d'actionnement (9).

11. Indicateur selon l'une quelconque des revendications précédentes, dans lequel ledit élément d'actionnement (1) comporte des moyens anti-retour (4) telle qu'une patte élastique, coopérant avec ledit élément rotatif de transmission (7) pour empêcher une rotation de celui-ci dans le sens opposé au sens de rotation lors d'un actionnement de l'indicateur.

12. Indicateur selon l'une quelconque des revendications précédentes, dans lequel ledit élément d'actionnement (1) comporte une première patte élastique (2) coopérant avec un premier engrenage d'actionnement (8) de l'élément rotatif de transmission (7), pour faire tourner ledit élément rotatif de transmission (7) dans le sens d'actionnement à chaque actionnement de l'indicateur, une seconde patte élastique anti-retour (4), coopérant avec ledit premier engrenage d'actionnement (8) de l'élément rotatif de transmission (7) pour éviter toute rotation dudit élément rotatif de transmission (7) en sens inverse audit sens d'actionnement, et une troisième patte élastique (5) coopérant avec une denture de positionnement (11) dudit second élément de comptage rotatif (10), pour assurer un positionnement angulaire précis dudit second élément de comptage rotatif (10) à chacune de ses rotations.

13. Indicateur selon l'une quelconque des revendications précédentes, dans lequel ledit élément d'actionnement (1) comporte une partie fixe par rapport au corps de l'indicateur (100) et une partie déformable, adaptée à coopérer avec une partie d'actionnement (50, 51) d'un dispositif de distribution de produit fluide (B), ladite partie d'actionnement (50, 51) étant mobile par rapport audit élément d'actionnement (1) sur une course d'actionnement.

14. Indicateur selon la revendication 13, dans lequel ladite partie déformable dudit élément d'actionnement comporte une première partie déformable ayant une première flexibilité, et une seconde partie déformable ayant une seconde flexibilité,ladite seconde flexibilité étant inférieure à ladite première flexibilité.

15. Indicateur selon la revendication 14, dans lequel ladite première partie déformable réalise l'actionnement de l'indicateur (A), et ladite seconde partie déformable plus rigide permet la poursuite de la course d'actionnement de ladite partie d'actionnement (50, 51) du dispositif de distribution de produit fluide, après actionnement dudit indicateur (A).

16. Indicateur selon l'une quelconque des revendications 13 à 15, dans lequel l'actionnement dudit indicateur (A) a lieu au tout début de la course d'actionnement dudit dispositif de distribution de produit fluide (B).

17. Indicateur selon l'une quelconque des revendications 13 à 16, dans lequel ladite partie d'actionnement du dispositif de distribution de produit fluide (B), qui coopère avec la partie déformable de l'élément d'actionnement (1), est un réservoir de produit fluide (51), ledit corps (100) de l'indicateur (A) étant fixe par rapport au corps (50) du dispositif de distribution de produit fluide (B).

18. Indicateur selon l'une quelconque des revendications 13 à 16, dans lequel ladite partie d'actionnement du dispositif de distribution de produit fluide (B), qui coopère avec la partie déformable de l'élément d'actionnement (1), est un corps (50) dudit dispositif de distribution de produit fluide (B), ledit corps (100) de l'indicateur (A) étant fixe par rapport à un réservoir de produit fluide (51).

19. Indicateur selon l'une quelconque des revendications précédentes, dans lequel ledit corps (100) de l'indicateur comporte une fenêtre de visualisation permettant d'afficher l'indication commune desdits premier et second éléments de comptage rotatifs (13, 10)

20. Dispositif d'inhalation de produit fluide (B), comportant un corps (50), un réservoir (51) mobile sur une course d'actionnement par rapport audit corps (50), et un embout d'inhalation (55), **caractérisé en ce qu'**il comporte en outre un indicateur (A) selon l'une quelconque des revendications précédentes.

## Patentansprüche

1. Dosen-Anzeigevorrichtung (A), die dazu dient, die Anzahl von Dosen anzuzeigen, die von einer Abgabevorrichtung (B) für ein fluidförmiges Produkt abgegeben worden sind oder noch abgegeben werden können, wobei die Anzeigevorrichtung einen Anzeigevorrichtungs-Körper (100) und lediglich vier verformbare und/oder verdrehbare Elemente, d.h. ein Betätigungselement (1), ein sich drehendes Übertragungselement (7), ein erstes sich drehendes Zählelement (13) und ein zweites sich drehendes Zählelement (10) umfasst, wobei das erste und das zweite Zählelement (13, 10) nebeneinander in derselben Ebene angeordnet sind und sich um parallele Rotationsachsen drehen, wobei das erste und das zweite Zählelement (13, 10) zusammenwirken, um eine gemeinsame Anzeige für jede Betätigung der Anzeigevorrichtung zu liefern, wobei das erste, sich drehende Zählelement (13) eine Scheibe ist, die eine obere und eine untere Oberfläche aufweist, wobei die obere Oberfläche erste Anzeigemittel umfasst, die im Bereich des äußeren Umfangsrandes dieser oberen Oberfläche angeordnet sind, und wobei die untere Oberfläche eine Verzahnung (16) umfasst, die mit einer Betätigungsverzahnung (9) zusammenwirkt, die an dem sich drehenden Übertragungselement (7) vorgesehen ist, wobei das erste, sich drehende Zählelement (13) einen Antriebszahn (15) aufweist, der ausgebildet ist, um bei jeder vollständigen Umdrehung des ersten, sich drehenden Zählelementes (13) mit einer zweiten Verzahnung (12) zusammen zu arbeiten, die an dem zweiten, sich drehenden Zählelement (10) vorgesehen ist.

2. Anzeigevorrichtung nach Anspruch 1, bei der die ersten Anzeigemittel Ziffern, insbesondere die Ziffern 0 bis 9 umfassen.

3. Anzeigevorrichtung nach einem der vorhergehenden Ansprüche, bei der das zweite, sich drehende Zählelement (10) eine Scheibe ist, die eine obere Oberfläche und eine untere Oberfläche aufweist, wobei die obere Oberfläche zweite Anzeigemittel umfasst, die im Bereich des äußeren Umfangsrandes dieser oberen Oberfläche angeordnet sind.

4. Anzeigevorrichtung nach Anspruch 3, bei der die zweiten Anzeigemittel Zahlen umfassen, insbesondere die Zahlen 00 bis 20.

5. Anzeigevorrichtung nach Anspruch 3 oder 4, bei der die untere Oberfläche des zweiten, sich drehenden Zählelementes (10) eine zweite Verzahnung (12) aufweist, die mit einem Antriebszahn (15) des ersten, sich drehenden Zählelementes (13) zusammenwirkt.

6. Anzeigevorrichtung nach einem der vorhergehenden Ansprüche, bei der das zweite, sich drehende Zählelement (10) eine Positionier-Verzahnung (11) aufweist, die mit einer elastischen Klaue (5) des Betätigungselementes (1) zusammenwirkt, insbesondere um eine genaue Positionierung des zweiten, sich drehenden Zählelementes (10) bei jeder seiner Drehungen sicherzustellen.

7. Anzeigevorrichtung nach einem der vorhergehenden Ansprüche, bei der das zweite, sich drehende Zählelement (10) Anschlagsmittel (19) umfasst, um die Betätigung der Anzeigevorrichtung (A) nach einer vorbestimmten Anzeige-Anzahl, insbesondere 200 zu blockieren.

8. Anzeigevorrichtung nach Anspruch 7, bei der die Anschlagsmittel (19) ein vorspringendes Profil wie zum Beispiel einen Zapfen (19) umfassen, das bzw. der mit dem zweiten, sich drehenden Zählelement (10) fest verbunden ist und mit einem festen Teil des Körpers (100) der Anzeigevorrichtung zusammenwirkt.

9. Anzeigevorrichtung nach einem der vorhergehenden Ansprüche, bei der das sich drehende Übertragungselement (7) eine erste Betätigungsverzahnung (8), die mit dem Betätigungselement (1) zusammenwirkt, und eine zweite Betätigungsverzahnung (9) aufweist, die mit einer ersten Verzahnung (16) des ersten, sich drehenden Zählelementes (13) zusammenwirkt.

10. Anzeigevorrichtung nach Anspruch 9, bei der das sich drehende Übertragungselement (7) eine Scheibe ist, die eine obere Oberfläche und eine untere Oberfläche besitzt, wobei die untere Oberfläche die besagte erste Betätigungsverzahnung (8) und die obere Oberfläche die besagte zweite Betätigungsverzahnung (9) umfasst.

11. Anzeigevorrichtung nach einem der vorhergehenden Ansprüche, bei der das Betätigungselement (1) eine Rückwärtsdrehung verhindernde Mittel (4) wie zum Beispiel eine elastische Klaue umfasst, die mit dem sich drehenden Übertragungselement (7) zusammenwirken, um eine Drehung desselben in einer Richtung zu verhindern, die der Drehrichtung bei der Betätigung der Anzeigevorrichtung entgegengesetzt ist.

12. Anzeigevorrichtung nach einem der vorhergehenden Ansprüche, bei der das Betätigungselement (1) eine erste elastische Klaue (2) umfasst, die mit einer ersten Betätigungsverzahnung (8) des sich drehenden Übertragungselementes (7) zusammenwirkt, um dieses sich drehende Übertragungselement (7) bei jeder Betätigung der Anzeigevorrichtung zu einer Drehung in der Betätigungsrichtung zu veranlassen, sowie eine zweite elastische Klaue (4) zur Verhinderung einer Rückwärtsdrehung, die mit der ersten Betätigungsverzahnung (8) des sich drehenden Übertragungselementes (7) zusammenwirkt, um jegliche Drehung des sich drehenden Übertragungselementes (7) in einer zur Betätigungsrichtung entgegengesetzten Richtung zu vermeiden, und eine dritte elastische Klaue (5), die mit einer Positionier-Verzahnung (11) des zweiten, sich drehenden Zählelementes (10) zusammenwirkt, um eine winkelmäßig genaue Positionierung des zweiten, sich drehenden Zählelementes (10) bei jeder seiner Drehungen sicherzustellen.

13. Anzeigevorrichtung nach einem der vorhergehenden Ansprüche, bei der das Betätigungselement (1) einen bezüglich des Körpers (100) der Anzeigevorrichtung festen Teil und einen verformbaren Teil umfasst, der geeignet ist, mit einem Betätigungsteil (50, 51) einer Abgabevorrichtung (B) für ein fluidförmiges Produkt zusammenzuwirken, wobei der Betätigungsteil (50, 51) bezüglich des Betätigungselementes (1) auf einem Betätigungsweg beweglich ist.

14. Anzeigevorrichtung nach Anspruch 13, bei der der verformbare Teil des Betätigungselementes (1) einen ersten verformbaren Teil aufweist, der eine erste Flexibilität besitzt, und einen zweiten verformbaren Teil, der eine zweite Flexibilität besitzt, wobei die zweite Flexibilität kleiner als die erste Flexibilität ist.

15. Anzeigevorrichtung nach Anspruch 14, bei welcher der erste verformbare Teil die Betätigung der Anzeigevorrichtung (A) bewirkt und der zweite, starrere verformbare Teil die Fortsetzung des Betätigungsablaufes des Betätigungsteils (50, 51) der Abgabevorrichtung für das fluidförmige Produkt nach der Betätigung der Anzeigevorrichtung (A) ermöglicht.

16. Anzeigevorrichtung nach einem der Ansprüche 13 bis 15, bei der die Betätigung der Anzeigevorrichtung (A) ganz am Anfang des Betätigungsablaufes der Abgabevorrichtung (B) für das fluidförmige Produkt erfolgt.

17. Anzeigevorrichtung nach einem der Ansprüche 13 bis 15, bei der der Betätigungsteil der Abgabevorrichtung (B) für das fluidförmige Produkt, der mit dem verformbaren Teil des Betätigungselementes (1) zusammenwirkt, ein Behälter (51) für das fluidförmige Produkt ist, wobei der Körper (100) der Anzeigevorrichtung (A) bezüglich des Körpers (50) der Abgabevorrichtung (B) für das fluidförmige Produkt feststehend ist.

18. Anzeigevorrichtung nach einem der Ansprüche 13 bis 16, bei welcher der Betätigungsteil der Abgabevorrichtung (B) für das fluidförmige Produkt, der mit dem verformbaren Teil des Betätigungselementes (1) zusammenwirkt, ein Körper (50) der Abgabevorrichtung (B) für das fluidförmige Produkt ist, wobei der Körper (100) der Anzeigevorrichtung (A) bezüglich eines Behälters (51) für das fluidförmige Produkt feststehend ist.

19. Anzeigevorrichtung nach einem der vorhergehenden Ansprüche, bei welcher der Körper (100) der Anzeigevorrichtung ein Sichtfenster aufweist, das es ermöglicht, die gemeinsame Anzeige des ersten sich drehenden und des zweiten sich drehenden Zählelementes (13, 10) sichtbar zu machen.

20. Inhalationsvorrichtung (B) für ein fluidförmiges Produkt, die einen Körper (50), einen auf einem Betätigungsweg bezüglich des Körpers (50) beweglichen Behälter (51) und einen Inhalations-Stutzen (55) umfasst, **dadurch gekennzeichnet, dass** sie weiterhin eine Anzeigevorrichtung (A) nach einem der vorhergehenden Ansprüche umfasst.

## Claims

1. A dose indicator (A) for indicating the number of doses that have been dispensed or that remain to be dispensed from a fluid dispenser device (B), the comprising an indicator body (100), and comprising only four deformable and/or rotary parts, namely a drive element (1), a rotary transmission element (7), a first rotary counter element (13), and a second rotary counter element (10), said first and second rotary counter elements (13, 10) being disposed side by side in a common plane and turning about parallel axes of rotation, said first and second counter elements (13, 10) co-operating to provide a common indication on each occasion the indicator is driven, said first rotary counter element (13) being a disk having a top surface and a bottom surface, said top surface including first indicator means located close to the outer peripheral edge of said top surface, and said bottom surface including a first gear (16) co-operating with a drive gear (9) provided on said rotary transmission element (7), said first rotary counter element (13) including a drive tooth (15) adapted to co-operate on each complete revolution of said first rotary counter element (13) with a second gear (12) provided on said second rotary counter element (10).

2. An indicator according to claim 1, wherein said first indicator means comprise digits, in particular the digits 0 to 9.

3. An indicator according to either preceding claim, wherein the second rotary counter element (10) is a disk having a top surface and a bottom surface, said top surface including second indicator means disposed close to the outer peripheral edge of said top surface.

4. An indicator according to claim 3, wherein said second indicator means comprise numbers, in particular the numbers 00 to 20.

5. An indicator according to claim 3 or claim 4, wherein said bottom surface of said second rotary counter element (10) includes a second gear (12) co-operating with a drive tooth (15) of said first rotary counter element (13).

6. An indicator according to any preceding claim, wherein said second rotary counter element (10) includes a set of positioning teeth (11) co-operating with a resilient tab (5) of said drive element (1), in particular to ensure that said second rotary counter element (10) is accurately positioned on each occasion it is turned.

7. An indicator according to any preceding claim, wherein said second rotary counter element (10) includes stop means (19) for preventing the indicator (A) being driven after a predetermined number of indications, in particular 200.

8. An indicator according to claim 7, wherein said stop means (19) comprise a projecting profile, such as a spur (19), secured to said second rotary counter element (10) and co-operating with a stationary portion of the indicator body (100).

9. An indicator according to any preceding claim, wherein said rotary transmission element (7) includes a first drive gear (8) co-operating with said drive element (1), and a second drive gear (9) co-operating with a first gear (16) of said first rotary counter element (13).

10. An indicator according to claim 9, wherein said rotary transmission element (7) is a disk having a top surface and a bottom surface, said bottom surface including said first drive gear (8) and said top surface including said second drive gear (9).

11. An indicator according to any preceding claim, wherein said drive element (1) includes anti-return means (4) such as a resilient tab co-operating with said rotary transmission element (7) to prevent it from turning in the direction opposite to the direction in which it turns when the indicator is driven.

12. An indicator according to any preceding claim, wherein said drive element (1) includes a first resilient tab (2) co-operating with a first drive gear (8) of the rotary transmission element (7) to cause said rotary transmission element (7) to turn in the drive direction each time the indicator is driven, an anti-return, second resilient tab (4) co-operating with said drive gear (8) of the rotary transmission element (7) to prevent any turning of said rotary transmission element (7) in a direction opposite to said drive direction, and a third resilient tab (5) co-operating with a set of positioning teeth (11) of said second rotary counter element (10) to ensure that said second rotary counter element (10) is angularly positioned accurately each time it is turned.

13. An indicator according to any preceding claim, wherein said drive element (1) includes a portion that is stationary relative to the indicator body (100), and a deformable portion adapted to co-operate with an actuation portion (50, 51) of a fluid dispenser device (B), said actuation portion (50, 51) being movable relative to said drive element (1) over an actuation stroke.

14. An indicator according to claim 13, wherein said deformable portion of said drive element includes a first deformable portion having first flexibility and a second deformable portion having second flexibility, said second flexibility being less than said first flexibility.

15. An indicator according to claim 14, wherein said first deformable portion drives the indicator (A), and said more-rigid second deformable portion allows the actuation stroke of said actuator portion (50, 51) of the fluid dispenser device to be continued after said indicator (A) has been driven.

16. An indicator according to any one of claims 13 to 15, wherein said indicator (A) is driven at the very beginning of the actuation stroke of said fluid dispenser device (B).

17. An indicator according to any one of claims 13 to 16, wherein said actuation portion of the fluid dispenser device (B) that co-operates with the deformable portion of the drive element (1) is a fluid reservoir (51), said body (100) of the indicator (A) being stationary relative to the body (50) of the fluid dispenser device (B).

18. An indicator according to any one of claims 13 to 16, wherein said actuation portion of the fluid dispenser device (B) that co-operates with the deformable portion of the drive element (1) is a body (50) of said fluid dispenser device (B), said body (100) of the indicator (A) being stationary relative to a fluid reservoir (51).

19. An indicator according to any preceding claim, wherein said indicator body (100) includes a viewing window for displaying the common indication of said first and second rotary counter elements (13, 10).

20. A fluid inhaler device (B) comprising a body (50), a reservoir (51) movable over an actuation stroke relative to said body (50), and an inhalation endpiece (55), the device being **characterized in that** it further includes an indicator (A) according to any preceding claim.
